# EUROPÄISCHE PATENTSCHRIFT

(11) **EP 3 215 486 B1**
(45) Veröffentlichungstag und Bekanntmachung des Hinweises auf die Patenterteilung: **09.01.2019**
(21) Anmeldenummer: 15791561.2
(22) Anmeldetag: 03.11.2015
(51) Int. Cl.: C07C 271/28, C07C 275/40, C08K 5/29, C08L 67/00, C08L 79/08

(54) **NEUE CARBODIIMIDE, VERFAHREN ZU DEREN HERSTELLUNG UND DEREN VERWENDUNG**
NEW CARBODIIMIDES, METHOD FOR THEIR MANUFACTURE AND USE OF SAME
NOUVEAU CARBODIIMIDE, SON PROCÉDÉ DE FABRICATION ET D'UTILISATION

(30) Priorität: 04.11.2014 EP 14191710
(43) Veröffentlichungstag der Anmeldung: 13.09.2017
(73) Patentinhaber: LANXESS Deutschland GmbH, 50569 Köln (DE)
(72) Erfinder: LAUFER, Wilhelm, 67158 Ellerstadt (DE); WENZEL, Volker, 68542 Heddesheim (DE)
(86) Internationale Anmeldenummer: PCT/EP2015/075612
(87) Internationale Veröffentlichungsnummer: WO 2016/071347

(56) Entgegenhaltungen:
- EP-A1- 0 628 541
- EP-A1- 1 138 706
- EP-A2- 0 460 481
- DE-A1- 2 248 751
- KR-A- 20110 085 073
- US-A- 2 941 983

## Beschreibung

Die Erfindung betrifft neue Carbodiimide mit endständigen Harnstoff- und/oder Urethangruppen, Verfahren zu deren Herstellung und deren Verwendung als Stabilisator in esterbasierten Polymeren vor allem in Folien zum Schutz vor hydrolytischem Abbau.

Carbodiimide haben sich in vielen Anwendungen bewährt, z.B. als Hydrolyseschutzmittel für Ester-basierte Thermoplasten, Polyole, Polyurethane, etc..

Bevorzugt werden hierfür sterisch gehinderte aromatische Monocarbodiimide eingesetzt. Bekannt ist in diesem Zusammenhang vor allem 2,6-Diisopropylphenyl-Carbodiimid. Diese Monocarbodiimide haben jedoch den Nachteil, selbst bei niedrigen Temperaturen flüchtig zu sein. Sie sind thermisch nicht stabil und können giftige, flüchtige Substanzen abspalten (Off-Gassing). Weitere Carbodiimide, wie in EP 0 628 541 A1 beschrieben, basieren auf speziellen Rohstoffen, die sehr teuer in der Anschaffung sind. Zudem haben diese bei Raumtemperatur hohe Viskositäten, die den Umgang mit diesen Carbodiimiden erschweren. Weiterhin ist in bestimmten PU-, PET-, oder PLA-Anwendungen deren Reaktivität und/oder deren stabilisierende Wirkung bei den standardmäßig eingesetzten Konzentrationen nicht ausreichend. Polymere Carbodiimide, die auf preiswerten Rohstoffen basieren, wie in DE-A 2248751 und US-A 2941983 beschrieben, sind nicht ausreichend sterisch gehindert und zeigen in den meisten esterbasierten Polymeren keine gute Hydrolyseschutzwirkung. Aus EP-A 0460481 sind NCO-Gruppen enthaltenden Carbodiimide bekannt. Diese haben allerdings den Nachteil, dass diese bereits bei Temperaturen unter 200 °C Isocyanate in solchen Konzentrationen freisetzen, die eine Verwendung in einer Reihe von Anwendungen wie z.B. in Folien oder im Automobilinnenraum ausschließt.

Es besteht daher die Aufgabe an neuen sterisch anspruchsvollen, leicht herstellbaren und leicht verarbeitbaren Carbodiimiden, welche eine hohe thermische Stabilität und ein sehr geringes Off-Gassing aufweisen und vor allem in Folienanwendungen eingesetzt werden können.

Überraschenderweise konnte diese Aufgabe durch den Einsatz von bestimmten aromatischen Carbodiimiden gelöst werden.

Gegenstand der vorliegenden Erfindung sind daher Carbodiimide mit endständigen Harnstoff- und oder Urethangruppen der Formel (I) 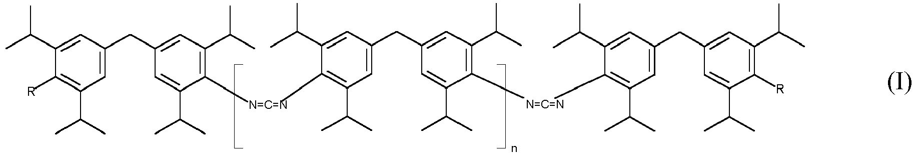 in der
- R gleich oder verschieden sein kann und ausgewählt ist aus der Gruppe der -NHCONHR^{I}-, -NHCONR^{I}R^{II}- und -NHCOOR^{III}-Reste, wobei R^{I} und R^{II} gleich oder verschieden sind und einem C₁ - C₂₂ - Alkyl-, C₆ - C₁₂ -Cycloalkyl-, C₆ - C₁₈ -Aryl- oder C₇ - C₁₈ -Aralkylrest entsprechen und R^{III} einem C₁ - C₂₂ - Alkyl-, bevorzugt C₁ - C₆ - Alkyl-, besonders bevorzugt Methyl-, Ethyl- oder i-Propyl-, C₆ - C₁₂ - Cycloalkyl-, bevorzugt C₆ -Cycloalkyl-, C₆ - C₁₈ -Aryl oder C₇ - C₁₈ -Aralkylrest, sowie einem ungesättigten Alkylrest (z.B. einen Oleylrest) mit 2 - 22, bevorzugt 12 - 20, besonders bevorzugt 16 - 18 Kohlenstoffatomen, oder ein Alkoxypolyoxyalkylenrest entspricht, und
- n = 0 bis 20, bevorzugt n = 1 bis 15 ist.

Der Carbodiimid-Gehalt (NCN-Gehalt, gemessen durch Titration mit Oxalsäure) der erfindungsgemäßen Carbodiimide liegt vorzugsweise bei 2 - 10 Gew.%.

Der Begriff C₇ - C₁₈ -Aralkylrest bedeutet, dass der Arylrest über eine Alkylfunktionalität an den Stickstoff in der Endgruppe R im Fall von R^{I} und R^{II} und im Fall von R^{III} an den Sauerstoff in der Endgruppe R gebunden ist.

Bevorzugte Alkoxypolyoxyalkylenreste sind Polyethylenglykolmonomethylether mit Molmassen von 200 - 600 g/mol, besonders bevorzugt von 350 - 550 g/mol.

Bevorzugt sind Carbodiimide der Formel (I) mit R = -NHCOOR^{III}-Rest mit R^{III} ein Alkoxypolyoxyalkylen oder ein ungesättigter Alkylrest mit 18 Kohlenstoffatomen ist, und n= 0 bis 20, bevorzugt n = 1 bis 10, besonders bevorzugt n = 2 bis 8, ganz besonders bevorzugt n = 3 bis 6.

Der Carbodiimid-Gehalt dieser bevorzugten Carbodiimide liegt vorzugsweise bei 2 - 8 Gew.%, besonders bevorzugt bei 3 - 6 Gew.%, ganz besonders bevorzugt bei 4 - 5 Gew.%.

Ebenfalls bevorzugt sind Carbodiimide der Formel (I) mit R = -NHCOOR^{III}, wobei R^{III} gleich C₁ - C₂₂ - Alkyl, bevorzugt C₁ - C₆ - Alkyl, besonders bevorzugt Methyl, Ethyl oder i-Propyl, C₆ - C₁₂ -Cycloalkyl, bevorzugt C₆ -Cycloalkyl ist und n = 0 bis 15, bevorzugt n = 1 bis 15, besonders bevorzugt n = 2 bis 10, ganz besonders bevorzugt n = 3 bis 8 bedeutet.

Der Carbodiimid-Gehalt dieser bevorzugten Carbodiimide liegt vorzugsweise bei 2 - 10 Gew.%, besonders bevorzugt bei 3 - 10 Gew.%, ganz besonders bevorzugt bei 4 - 8 Gew.%.

Die erfindungsgemäßen Carbodiimide weisen darüber hinaus vorzugsweise mittlere Molmassen (Mw) von 1000 - 10000 g/mol, bevorzugt 2000 - 8000 g/mol, besonders bevorzugt 3000 - 6000 g/mol auf.

Weiterhin sind Carbodiimide bevorzugt, die eine Polydispersität D = Mw/Mn von 1,2 - 2,5, besonders bevorzugt von 1,4 - 1,8 aufweisen.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Gegenstand der vorliegenden Erfindung ist zudem die Herstellung der erfindungsgemäßen Carbodiimide durch die Carbodiimidisierung von aromatischen Diisocyanaten der Formel (II) 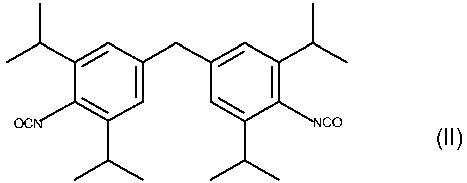 unter Abspaltung von Kohlendioxid bei Temperaturen von 80 °C bis 200 °C in Gegenwart von Katalysatoren und gegebenenfalls Lösungsmittel und der anschließenden Endfunktionalisierung der freien NCO-Gruppen mit primären oder sekundären Aminen und/oder Alkoholen und/oder Alkoxypolyoxyalkylenalkoholen.

Die für die Herstellung der Diisocyanate notwendigen aromatischen Diamine können - wie dem Fachmann bekannt- über eine Friedel-Crafts-Alkylierung der entsprechenden 4,4'- Diaminodiphenylmethan mit Propen hergestellt werden. Bei den aromatischen Diaminen handelt es sich um handelsübliche Verbindungen, die z.B. bei der Firma Lonza AG unter dem Handelsnamen Lonzacure® M-DIPA erhältlich sind.

Anschließend werden diese Diamine mit Phosgen zum entsprechenden Diisocyanat der Formel (II), M-DIPI, umgesetzt.

Zur Herstellung der erfindungsgemäßen Carbodiimide können die Diisocyanate der Formel (II), M-DIPI, bei erhöhter Temperaturen, vorzugsweise Temperaturen von 80 - 200 °C, besonders bevorzugt von 100 bis 180°C, ganz besonders bevorzugt von 140 - 160°C, zweckmäßigerweise in Gegenwart von Katalysatoren unter Kohlendioxidabspaltung kondensiert werden. Hierfür geeignete Verfahren werden beispielsweise beschrieben in DE-A 1130594 und DE-A 1156401.

Als Katalysatoren für die Herstellung der Verbindungen der Formel (I) sind in einer Ausführungsform der Erfindung Phosphorverbindungen bevorzugt. Als Phosphorverbindungen werden vorzugsweise Phospholenoxide, Phospholidine oder Phospholinoxide sowie die entsprechenden Phospholensulfide verwendet. Ferner können als Katalysatoren tertiäre Amine, basisch reagierende Metallverbindungen, Alkali-, Erdalkalioxide oder -Hydroxide, -Alkoholate oder -Phenolate, Carbonsäuremetallsalze und nicht basische Organometallverbindungen verwendet werden.

Die Carbodiimidisierung kann sowohl in Substanz als auch in einem Lösemittel durchgeführt werden. Als Lösemittel werden vorzugsweise Alkylbenzole, Paraffinöle, Polyethylenglykoldimethylether, Ketone oder Lactone eingesetzt.

Wenn die Reaktionsmischung den gewünschten Gehalt an NCO-Gruppen, entsprechend einem mittleren Kondensationsgrad von n = 0 bis 20, bevorzugt n = 1 bis 10 besitzt wird die Polycarbodiimidisierung üblicherweise gestoppt.

Anschließend werden die freien endständigen Isocyanatgruppen der Carbodiimide mit primären oder sekundären aliphatischen und/oder aromatischen Aminen, Alkoholen und/oder Alkoxypolyoxyalkylenalkoholen vorzugsweise in einem geringem Überschuss an -NH-, -NH₂- und/oder -OH-Gruppen, gegebenenfalls in Anwesenheit eines dem Fachmann bekannten PU-Katalysators, vorzugsweise tert. Aminen oder Organozinn-Verbindungen, besonders bevorzugt DBTL (Dibutylzinndilaurat oder DOTL (Dioctylzinndilaurat), abreagiert. Das Stoffmengen-Verhältnis von Aminen, Alkoholen und/oder Alkoxypolyoxyalkylenalkoholen zu Carbodiimiden liegt vorzugsweise bei 1,005 - 1,05 : 1, besonders bevorzugt bei 1,01 - 1,03 : 1, bezogen auf die vorhandenen N=C=O-Gruppen.

Als Alkohole sind Ethanol und Cyclohexanol bevorzugt.

In einer weiteren Ausführungsform der vorliegenden Erfindung wird zur Unterbrechung der Carbodiimidisierung die Temperatur der Reaktionsmischung auf 50 - 120 °C, bevorzugt 60 - 100 °C, besonders bevorzugt auf 80 - 90 °C reduziert und gegebenenfalls nach Zugabe eines Lösemittels, bevorzugt ausgewählt aus der Gruppe der Alkylbenzole, besonders bevorzugt Toluol, werden die freien endständigen Isocyanatgruppen der Carbodiimide mit aliphatischen und/oder aromatischen Aminen, Alkoholen und/oder Alkoxypolyoxyalkylenalkoholen vorzugsweise in einem geringem Überschuss an -NH-, -NH₂- und/oder -OH-Gruppen, gegebenenfalls in Anwesenheit eines dem Fachmann bekannten PU-Katalysators, vorzugsweise tert. Aminen oder Organozinn-Verbindungen, besonders bevorzugt DBTL (Dibutylzinndilaurat oder DOTL (Dioctylzinndilaurat), abreagiert. Das Stoffmengen-Verhältnis von Aminen, Alkoholen und/oder Alkoxypolyoxyalkylenalkoholen zu Carbodiimiden liegt vorzugsweise bei 1,005 - 1,05 : 1, besonders bevorzugt bei 1,01 - 1,03 : 1 bezogen auf die vorhandenen N=C=O-Gruppen.

Nach vollständiger Reaktion wird der Katalysator und gegebenenfalls das Lösemittel vorzugsweise bei Temperaturen von 80 - 200°C unter verminderten Druck abdestilliert.

Als Alkohole sind Ethanol und Cyclohexanol bevorzugt.

Gegenstand der vorliegenden Erfindung ist zudem ein weiteres Verfahren zur Herstellung der erfindungsgemäßen Carbodiimide durch eine partielle, vorzugsweise < 50 %ige Endfunktionalisierung der freien NCO-Gruppen mit primären oder sekundären Aminen oder Alkoholen und/oder Alkoxypolyoxyalkylenalkoholen in den aromatischen Diisocyanaten der Formel (II) 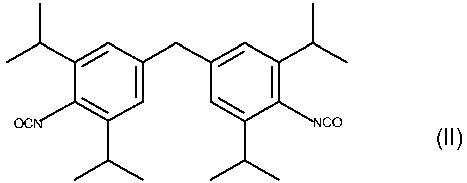 und anschließende Carbodiimidisierung unter Abspaltung von Kohlendioxid bei Temperaturen von 80 °C bis 200 °C in Gegenwart von Katalysatoren und gegebenenfalls Lösungsmittel.

Vorzugsweise werden die erfindungsgemäßen Carbodiimide nach ihrer Herstellung gereinigt. Die Reinigung der Rohprodukte kann destillativ und/oder mittels Extraktion mit Lösemitteln und/oder mittels Umkristallisation in Lösemitteln erfolgen. Als geeignete Lösemittel für die Aufreinigung können vorzugsweise Polyethylenglykoldimethylether, Alkylbenzole, Paraffinöle, Alkohole, Ketone oder Ester eingesetzt werden. Dabei handelt es sich um handelsübliche Lösemittel.

Ein weiterer Gegenstand der vorliegenden Erfindung ist zudem ein bevorzugtes Verfahren zur Herstellung der erfindungsgemäßen Carbodiimide der Formel (I) mit R = -NHCOOR^{III}, wobei R^{III} gleich C₁ - C₂₂ - Alkyl, bevorzugt C₁ - C₆ - Alkyl, besonders bevorzugt Methyl, Ethyl oder i-Propyl, C₆ - C₁₂ -Cycloalkyl, besonders bevorzugt C₆-Cycloalkyl ist und n = 0 bis 20, bevorzugt n = 1 bis 15, besonders bevorzugt n = 2 - 10, ganz besonders bevorzugt n = 3 bis 8 ist, bei dem die Schmelze nach der Carbodiimidisierung und gegebenenfalls Aufreinigung bevorzugt auf Pastillierbändern pastilliert wird. Dabei sind gängige Pastilliersysteme ebenso wie gängige Granuliersysteme einsetzbar. Diese sind z.B. erhältlich bei der Firma Sandvik Holding GmbH oder der Firma GMF Gouda.

Ganz besonders geeignet sind die erfindungsgemäßen Carbodiimide der Formel (I) mit R = -NHCOOR^{III}, wobei R^{III} Cyclohexyl ist.

Gegenstand der vorliegenden Erfindung ist weiterhin eine Zusammensetzung enthaltend
- mindestens ein esterbasiertes Polymer,
   und
- mindestens ein erfindungsgemäßes Carbodiimid der Formel (I).

Bei den esterbasierten Polymeren handelt es sich vorzugsweise um Polyesterpolyole, esterbasierte thermoplastische Polyurethane, esterbasierte Polyurethan-Elastomere oder -Schäume, Polyethylenterephthalat (PET), Polybutylenterephthalat (PBT), Polytrimethylenterephthalat (PTT), Copolyester, wie vorzugsweise modifizierte Polyester aus Cyclohexandiol und Terephthalsäure (PCTA), thermoplastische Polyester Elastomere (TPE E), Ethylenvinylacetat (EVA), Polymilchsäure (PLA) und/oder PLA-Derivate, Polyhydroxyalkanoate (PHA), Polybutylenadipat-Terephthalat (PBAT), Polybutylensuccinat (PBS), in Polyamid (PA) wie z. B. Polyamid 6, 6.6, 6.10, 6.12, 10, 11, 12 oder in Blends, wie vorzugsweise PA/PET- oder PHA/PLA-Blends. Hierbei handelt es sich um kommerziell erhältliche Polymere.

Die Konzentration der erfindungsgemäßen Carbodiimide der Formel (I) in der erfindungsgemäßen Zusammensetzung beträgt vorzugsweise 0,1 - 10 Gew.%, bevorzugt 1 - 5 Gew.%, besonders bevorzugt 1 - 3 Gew.%.

Gegenstand der vorliegenden Erfindung ist zudem ein Verfahren zur Herstellung der erfindungsgemäßen Zusammensetzung, bei dem die erfindungsgemäßen Carbodiimide der Formel (I) mit R = -NHCOOR^{III}, mit R^{III} gleich C₁ - C₂₂ - Alkyl, bevorzugt C₁- C₆ - Alkyl, besonders bevorzugt Methyl, Ethyl oder i-Propyl, C₆ - C₁₂-Cycloalkyl, bevorzugt C₆ -Cycloalkyl ist und n = 0 bis 20, bevorzugt n = 1 bis 15, besonders bevorzugt n = 2 bis 10, ganz besonders bevorzugt n = 3 bis 8 zu den esterbasierten Polymeren ausgewählt aus der Gruppe, umfassend Polyethylenterephthalat (PET), Polybutylenterephthalat (PBT), Polytrimethylenterephthalat (PTT), thermoplastische Polyurethane (TPU), Copolyester, wie das modifizierte Polyester aus Cyclohexandiol und Terephthalsäure (PCTA), thermoplastische Polyester Elastomere (TPE E), Ethylenvinylacetat (EVA), Polybutylenadipat-Terephthalat (PBAT), Polybutylensuccinat (PBS), Polymilchsäure (PLA) und/oder PLA-Derivate, Polyhydroxyalkanoate (PHA), in Polyamid (PA) wie z. B.

Polyamid 6, 6.6, 6.10, 6.12, 10, 11, 12 oder in Blends, wie z.B. PA/PET- oder PHA/PLA-Blends, mittels Feststoffdosieraggregaten zugegeben werden.

Feststoffdosieraggregate im Sinne der Erfindung sind vorzugsweise: Ein-, Zwei- und Mehrwellenextruder, kontinuierlich arbeitenden Co-Kneter (Typ Buss) und diskontinuierlich arbeitenden Kneter, z.B. Typ Banbury und andere in der Polymerindustrie üblichen Aggregate.

Bei den Polyesterpolyolen als esterbasierte Polymere handelt es sich vorzugsweise um langkettige Verbindungen, die vorzugsweise ein Molekulargewicht (in g/mol) von bis zu 2000, bevorzugt zwischen 500 - 2000 und besonders bevorzugt zwischen 500 - 1000, aufweisen.

Der Begriff Polyesterpolyole im Sinne der Erfindung umfasst dabei sowohl langkettige Diole als auch Triole, wie auch Verbindungen mit mehr als drei Hydroxylgruppen je Molekül.

Vorteilhaft ist es, wenn das Polyesterpolyol eine OH-Zahl von bis zu 200, vorzugsweise zwischen 20 und 150 und besonders bevorzugt zwischen 50 und 115, aufweist. Insbesondere eignen sich Polyesterpolyole, die Reaktionsprodukte von verschiedenen Polyolen mit aromatischen oder aliphatischen Dicarbonsäuren und/oder Polymere von Lactonen sind.

Bei den im Sinne der Erfindung eingesetzten Polyesterpolyolen handelt es sich um handelsübliche Verbindungen, die bei der Firma Bayer MaterialScience AG unter dem Handelsnamen Baycoll® oder Desmophen® erhältlich sind.

Gegenstand der vorliegenden Erfindung ist zudem die Verwendung der erfindungsgemäßen Carbodiimide in esterbasierten Polyolen, Polyethylenterephthalat (PET), Polybutylenterephthalat (PBT), Polytrimethylenterephthalat (PTT), Copolyestern, wie das modifizierte Polyester aus Cyclohexandiol und Terephthalsäure (PCTA), thermoplastischen Polyester Elastomeren (TPE E), Ethylenvinylacetat (EVA), Polymilchsäure (PLA) und/oder PLA-Derivaten, Polyhydroxyalkanoaten (PHA), Polybutylenadipat-Terephthalat (PBAT), Polybutylensuccinat (PBS), in Polyamid (PA) wie z. B. Polyamid 6, 6.6, 6.10, 6.12, 10, 11, 12 oder in Blends, wie z.B. PA/PET- oder PHA/PLA-Blends, in thermoplastischen Polyurethanen (TPU), in Polyurethan-Elastomeren, in PU-Klebstoffen, in PU-Gießharzen, in PU-Schäumen oder in PU-Beschichtungen für Holz, Leder, Kunstleder und Textilien als Schutz gegen hydrolytischen Abbau.

Gegenstand der vorliegenden Erfindung sind zudem Folien enthaltend mindestens einen Polyester, ausgewählt aus der Gruppe Polyethylenterephthalat (PET), Ethylenvinylacetat (EVA), Polyethylennaphthalat (PEN), Polybutylenterephthalat (PBT), Polytrimethylene-terephthalat (PTT) und/oder Polycyclohexandimethanol-terephthalat (PCT) und 1,0 - 3,0 Gew.% mindestens ein erfindungsgemäßes Carbodiimid, bezogen auf den Polyester.

Die Herstellung der Folie erfolgt vorzugsweise durch Vermischen des erfindungsgemäßen Carbodiimids oder gegebenenfalls eines Carbodiimid-Masterbatches mit dem Polyester in der Schmelze und anschließender Schmelzextrusion, siehe auch EP-A 2262000.

Für die Schmelzextrusion sind folgenden Geräte einsetzbar: Einwellen-, Doppelwellen- oder Mehrwellenextruder, Planetenextruder, Kaskadenextruder, kontinuierlich arbeitende Co-Kneter (Typ Buss) und diskontinuierlich arbeitende Kneter, z.B. Typ Banbury und andere in der Polymerindustrie übliche Aggregate.

Die Folien können dabei in beliebiger Dicke hergestellt werden. Bevorzugt sind jedoch Schichtdicken zwischen 25 und 300 Mikrometer.

Gegenstand der vorliegenden Erfindung ist zudem die Verwendung der erfindungsgemäßen Folie in Solarzellen und dort vorzugsweise zum Abdichten und damit zum Schutz vor Umgebungseinflüssen, wie z.B. Feuchtigkeit und dem Eindringen von Fremdkörpern.

Ein weiterer Gegenstand der Erfindung sind auch Formmassen aus Polyamid (PA) enthaltend 1,0 - 3,0 Gew.% des erfindungsgemäßen Carbodiimids, bezogen auf das Polyamid, und gegebenenfalls weitere Additive und Füll- und/oder Verstärkungsstoffe, vorzugsweise Glasfaser.

Erfindungsgemäss bevorzugte Polyamide sind teilkristalline oder amorphe Polyamide, die ausgehend von Diaminen und Dicarbonsäuren und/oder Lactamen mit wenigstens 5 Ringgliedern oder entsprechenden Aminosäuren hergestellt werden können. Als Edukte kommen bevorzugt aliphatische und/oder aromatische Dicarbonsäuren, besonders bevorzugt Adipinsäure, 2,2,4-Trimethyladipinsäure, 2,4,4-Trimethyladipinsäure, Azelainsäure, Sebazinsäure, Isophthalsäure, Terephthalsäure, aliphatische und/oder aromatische Diamine, besonders bevorzugt Tetramethylendiamin, Pentamethylendiamin, Hexamethylendiamin, 1,9-Nonandiamin, 2,2,4- und 2,4,4-Trimethylhexamethylendiamin, die Isomeren Diaminodicyclohexylmethane, Diaminodicyclohexylpropane, Bisaminomethyl-cyclohexan, Phenylendiamine, Xylylendiamine, Aminocarbonsäuren, insbesondere Aminocapronsäure, oder die entsprechenden Lactame in Betracht. Copolyamide aus mehreren der genannten Monomeren sind eingeschlossen.

Besonders bevorzugt ist Polyamid 6 oder Polyamid 6.6, ganz besonders bevorzugt wird Polyamid 6.6 eingesetzt.

Es können außerdem Anteile von rezyklierten Polyamid-Formmassen und / oder Faserrezyklaten enthalten sein.

Die Polyamide weisen vorzugsweise eine relative Viskosität von 2,3 bis 4,0 besonders bevorzugt von 2,7 bis 3,5 auf, wobei die relative Viskosität an einer 1 gew.-%igen Lösung in m-Kresol bei 25°C bestimmt bzw. gemessen werden kann.

Additive im Sinne der vorliegenden Erfindung sind Stabilisatoren, Antistatika, Fliesshilfsmittel, Entformungsmittel, Brandschutzadditive, Emulgatoren, Nukleierungsmittel, Weichmacher, Gleitmittel, Farbstoffe, Pigmente, Verzweiger, Kettenverlängerer oder Additive zur Erhöhung der elektrischen Leitfähigkeiten. Die Additive können alleine oder in Mischung bzw. in Form von Masterbatchen eingesetzt werden.

Als Füllstoff oder Verstärkungsstoff können einzeln oder auch als Mischung aus zwei oder mehr unterschiedlichen Füllstoffen und/oder Verstärkungsstoffen eingesetzt werden. Bevorzugt werden Füllstoffe und/oder Verstärkungsstoffe der Reihe Talk, Glimmer, Silikat, Quarz, Titandioxid, Wollastonit, Kaolin, amorphe Kieselsäuren, Magnesiumcarbonat, Kreide, Feldspat, Bariumsulfat, Glaskugeln und/oder faserförmige Füllstoffe und/oder Verstärkungsstoffe auf der Basis von Kohlenstofffasern und/oder Glasfasern eingesetzt.. Besonders bevorzugt werden mineralische teilchenförmige Füllstoffe auf der Basis von Talk, Glimmer, Silikat, Quarz, Titandioxid, Wollastonit, Kaolin, amorphe Kieselsäuren, Magnesiumcarbonat, Kreide, Feldspat, Bariumsulfat und/oder Glasfasern eingesetzt.

Ganz besonders bevorzugt werden mineralische teilchenförmige Füllstoffe auf der Basis von Talk, Wollastonit, Kaolin und/oder Glasfasern eingesetzt.

Insbesondere bevorzugt werden ferner auch nadelförmige mineralische Füllstoffe eingesetzt. Unter nadelförmigen mineralischen Füllstoffen wird erfindungsgemäß ein mineralischer Füllstoff mit stark ausgeprägtem nadelförmigen Charakter verstanden. Als Beispiel seien nadelförmige Wollastonite genannt. Bevorzugt weist dieses Mineral ein Länge : Durchmesser - Verhältnis von 2:1 bis 35:1, besonders bevorzugt von 3:1 bis 19:1, insbesondere bevorzugt von 4:1 bis 12:1 auf. Die mittlere Teilchengröße der erfindungsgemäßen nadelförmigen Mineralien liegt bevorzugt bei kleiner 20 µm, besonders bevorzugt bei kleiner 15 µm, insbesondere bevorzugt bei kleiner 10 µm, bestimmt mit einem CILAS GRANULOMETER.

Der eingesetzte Füllstoff und/oder Verstärkungsstoff kann auch Oberflächen-modifiziert sein, wie beschrieben in EP-A 2562219.

Die erfindungsgemäß insbesondere bevorzugt einzusetzenden Glasfasern können entweder eine kreisförmige Querschnittsfläche und einen Filament-Durchmesser von 6 bis 18 µm, bevorzugt zwischen 9 und 15 µm, oder eine flache Gestalt und nichtkreisförmige Querschnittsfläche, deren Hauptquerschnittsachse eine Breite im Bereich von 6 - 40 µm und deren Nebenquerschnittsachse eine Breite im Bereich von 3 - 20 µm besitzt, aufweisen. Die Glasfaser wird bevorzugt ausgewählt aus der Gruppe der E-Glasfasern, A-Glasfasern, C-Glasfasern, D-Glasfasern, S-Glasfasern und / oder R-Glasfasern.

Die Glasfasern können als Endlosfasern oder als geschnittene oder gemahlene Glasfasern zugesetzt werden. Die Fasern können mit einem geeigneten Schlichtesystem, enthaltend u.a. bevorzugt Haftvermittler insbesondere auf Silanbasis ausgerüstet sein, wie beispielsweise beschrieben in EP-A 2562219.

Für die Ausrüstung der Füllstoffe werden bevorzugt Silanverbindungen im Allgemeinen in Mengen von 0,05 bis 2 Gew.-%, vorzugsweise 0,25 bis 1,5 Gew.-% und insbesondere 0,5 bis 1 Gew.-% bezogen auf den mineralischen Füllstoff zur Oberflächenbeschichtung eingesetzt.

Die teilchenförmigen Füllstoffe können bedingt durch die Verarbeitung zur Formmasse bzw. Formkörper in der Formmasse bzw. im Formkörper einen kleineren d₉₇- bzw. d₅₀-Wert aufweisen, als die ursprünglich eingesetzten Füllstoffe. Die Glasfasern können bedingt durch die Verarbeitung zur Formmasse bzw. Formkörper in der Formmasse bzw. im Formkörper kürzere Längenverteilungen als ursprünglich eingesetzt aufweisen.

Die nachfolgenden Beispiele dienen der Erläuterung der Erfindung, ohne dabei limitierend zu wirken.

### Ausführungsbeispiele

Getestet wurden:
1) CDI (A): ein Carbodiimid gemäß Formel (I) mit R = NCO und n > 20, mit einem NCN-Gehalt von ca. 11 Gew.% und mit einem NCO-Gehalt von < 1 Gew.%, Vergleich.
2) CDI (B): ein Carbodiimid der Formel (I) mit R = -NHCOOR^{III} und R^{III} = Cyclohexyl, mit einem NCN-Gehalt von ca. 6 Gew.% und n = ca. 3, erfindungsgemäß.

### Herstellung des Carbodiimides CDI (A), Vergleich

In einem ausgeheizten und mit Stickstoff befüllten 250 ml Vierhalskolben wurden unter Stickstoffstrom 92 g des Diisocyanats der Formel (II), M-DIPI, vorgelegt. Nach Zugabe von 50 mg 1-Methyl-phospholenoxid wurde auf 160 °C erwärmt. Im Anschluss wurde bei 160 °C unter Abspaltung von Kohlendioxid solange carbodiimidisiert bis ein NCO-Gehalt von ca. 1 Gew.% erreicht worden war. Das erhaltene Produkt war bei 160° nicht mehr rührbar. Die Viskosität bei 140 °C betrug > 1000 Pas, so dass keine Pastillierung möglich war.

### Herstellung des erfindungsgemäßen Carbodiimides CDI (B)

In einem ausgeheizten und mit Stickstoff befüllten 250 ml Vierhalskolben wurden unter Stickstoffstrom 92 g des Diisocyanats der Formel (II), M-DIPI, vorgelegt. Nach Zugabe von 50 mg 1-Methyl-phospholenoxid wurde auf 160 °C erwärmt. Im Anschluss wurde bei 160 °C unter Abspaltung von Kohlendioxid solange carbodiimidisiert bis ein NCO-Gehalt von ca. 6 Gew.% erreicht worden war. Schließlich wurde die Reaktionsmischung auf ca. 90 - 100°C gekühlt und die endständigen NCO-Gruppen mit Cyclohexanol in Toluol als Lösungsmittel abreagiert (freier NCO-Gehalt < 0,1 %). Nach der destillativen Abtrennung des Toluols wurde ein Produkt mit einem NCN-Gehalt von ca. 6 Gew.% erhalten. Dieses war bei 160° noch sehr gut rührbar und konnte einwandfrei pastilliert werden. Die Viskosität bei 140 °C betrug < 10 Pas. Die mittlere Molmasse betrug ca. 3000 g/mol.

Die Ergebnisse zeigen, dass die erfindungsgemäßen Carbodiimide im Vergleich zum Stand der Technik handhabbare Viskositäten in der Schmelze aufzeigen und dadurch überhaupt eine großtechnische Herstellung möglich ist. Darüber hinaus haben diese den Vorteil, dass sie auf einer günstigeren Rohstoffbasis basieren.

## Patentansprüche

1. Carbodiimide mit endständigen Harnstoff- und/oder Urethangruppen der Formel (I) 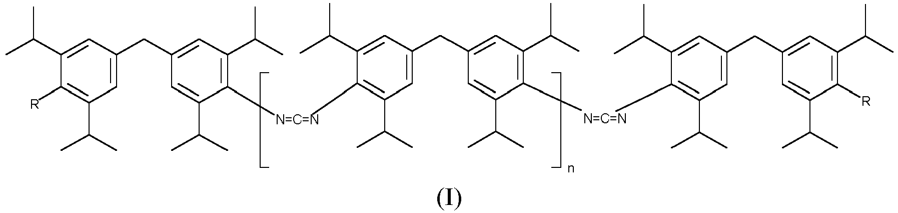
- in der R gleich oder verschieden sein kann und ausgewählt ist aus der Gruppe der -NHCONHR^{I}-, -NHCONR^{I}R^{II}- und -NHCOOR^{III}-Reste, wobei R^{I} und R^{II} gleich oder verschieden sind und einem C₁ - C₂₂ - Alkyl-, C₆ - C₁₂-Cycloalkyl-, C₆ - C₁₈-Aryl- oder C₇ - C₁₈ -Aralkylrest entsprechen und R^{III} einem C₁ - C₂₂ - Alkyl-, C₆ - C₁₂ -Cycloalkyl-, C₆ - C₁₈ -Aryl- oder C₇ - C₁₈ -Aralkylrest, oder einem ungesättigten Alkylrest mit 2-22, bevorzugt 12-20, besonders bevorzugt 16-18 Kohlenstoffatomen, oder einem Alkoxypolyoxyalkylenrest entspricht, und
- n = 0 bis 20, bevorzugt n = 1 bis 15 bedeutet.

2. Carbodiimide nach Anspruch 1, **dadurch gekennzeichnet, dass** R ein -NHCOOR^{III}-Rest mit R^{III} ein Alkoxypolyoxyalkylen oder ein ungesättigter Alkylrest mit 18 Kohlenstoffatomen ist, und n = 0 bis 20, bevorzugt n = 1 bis 10, besonders bevorzugt n = 2 bis 8, ganz besonders bevorzugt n = 3 bis 6 ist.

3. Carbodiimide nach Anspruch 1, **dadurch gekennzeichnet, dass** R ein -NHCOOR^{III}-Rest ist, wobei R^{III} C₁ - C₂₂ - Alkyl, bevorzugt C₁ - C₆ - Alkyl, besonders bevorzugt Methyl, Ethyl oder i-Propyl, C₆ - C₁₂ -Cycloalkyl, ganz besonders bevorzugt C₆ -Cycloalkyl ist, und n = 0 bis 15, bevorzugt n = 1 bis 15, besonders bevorzugt n = 2 bis 10, ganz besonders bevorzugt n = 3 bis 8 bedeutet.

4. Carbodiimide nach Anspruch 2, **dadurch gekennzeichnet, dass** der NCN-Gehalt im Carbodiimid 2 - 8 Gew.-%, bevorzugt 3 - 6 Gew.-%, besonders bevorzugt 4 - 5 Gew.% beträgt.

5. Carbodiimide nach Anspruch 3, **dadurch gekennzeichnet, dass** der NCN-Gehalt im Carbodiimid 2 - 10 Gew.-%, bevorzugt 3 - 10 Gew.-%, besonders bevorzugt bei 4 - 8 Gew.%, beträgt.

6. Carbodiimide nach einem der Ansprüche 1 bis 5, **dadurch gekennzeichnet, dass** diese mittlere Molmassen (Mw) von 1000 - 10000 g/mol, bevorzugt 2000 - 8000 g/mol, besonders bevorzugt 3000 - 6000 g/mol aufweisen.

7. Verfahren zur Herstellung der Carbodiimide nach einem der Ansprüche 1 bis 6 **dadurch gekennzeichnet, dass** Diisocyanate der Formel (II) 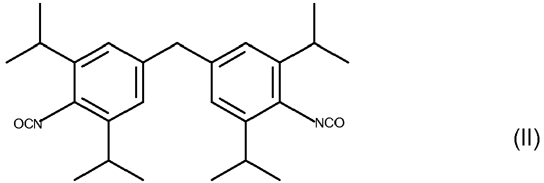 unter Abspaltung von Kohlendioxid bei Temperaturen von 80 °C bis 200 °C in Gegenwart von Katalysatoren und gegebenenfalls Lösungsmittel carbodiimidisiert werden und anschließend die freien NCO-Funktionalitäten mit primären oder sekundären Aminen und/oder Alkoholen endfunktionalisiert werden.

8. Verfahren zur Herstellung der Carbodiimide nach einem der Ansprüche 1 bis 6 **dadurch gekennzeichnet, dass** zuerst eine partielle, vorzugsweise < 50% ige Endfunktionalisierung der freien NCO-Gruppen mit primären oder sekundären Aminen und/oder Alkoholen und/oder Alkoxypolyoxyalkylenalkoholen in den aromatischen Diisocyanaten der Formel (II) 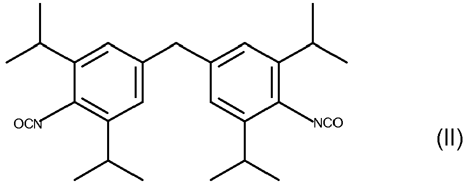 durchgeführt wird und anschließend eine Carbodiimidisierung unter Abspaltung von Kohlendioxid bei Temperaturen von 80 °C bis 200 °C in Gegenwart von Katalysatoren und gegebenenfalls Lösungsmittel erfolgt.

9. Verfahren zur Herstellung der Carbodiimide nach Anspruch 3, **dadurch gekennzeichnet, dass** die Schmelze nach der Herstellung auf Pastillierbändern pastilliert wird.

10. Zusammensetzung enthaltend
- mindestens ein esterbasiertes Polymer, vorzugsweise ausgewählt aus der Gruppe der Polyesterpolyole, der esterbasierten thermoplastischen Polyurethane, der Polyurethan-Elastomere, der PU-Klebstoffe, der PU-Gießharzen, der Polyamide (PA), der Polyethylenterephthalate (PET), der Polybutylenterephthalate (PBT), der Polytrimethylenterephthalate (PTT), Copolyester, der thermoplastischen Polyester-Elastomere (TPE E), der Ethylenvinylacetate (EVA), der Polymilchsäuren (PLA), der Polybutylenadipat-Terephthalate (PBAT), der Polybutylensuccinate (PBS), der PLA-Derivate und/oder der Polyhydroxyalkanoate (PHA)
und
- mindestens ein Carbodiimid nach einem der Ansprüche 1 bis 6.

11. Zusammensetzung nach Anspruch 10 **dadurch gekennzeichnet, dass** die Konzentration des Carbodiimides 0,1 - 10 Gew.%, bevorzugt 1 - 5 Gew.%, besonders bevorzugt 1 - 3 Gew.% beträgt.

12. Verfahren zur Herstellung der Zusammensetzungen nach Anspruch 10 oder 11, **dadurch gekennzeichnet, dass** Carbodiimide nach Anspruch 3 zu den esterbasierten Polymeren ausgewählt aus der Gruppe, umfassend Polyethylenterephthalat (PET), Polybutylenterephthalat (PBT), Polytrimethylenterephthalat (PTT), Polyamide (PA), thermoplastische Polyurethane (TPU), Copolyester, modifizierte Polyester aus Cyclohexandiol und Terephthalsäure (PCTA), thermoplastische Polyester Elastomere (TPE E), Ethylenvinylacetat (EVA), Polymilchsäure (PLA), Polybutylenadipat-Terephthalate (PBAT), Polybutylensuccinate (PBS), PLA-Derivate und/oder Polyhydroxyalkanoate (PHA), mittels Feststoffdosieraggregat zugegeben werden.

13. Verwendung der Carbodiimide nach einem der Ansprüche 1 bis 6 in esterbasierten Polyolen, in Polyamiden (PA), in Polyethylenterephthalat (PET), in Polybutylenterephthalat (PBT), in Polytrimethylenterephthalat (PTT), in Copolyestern, in thermoplastischen Polyester-Elastomeren (TPE E), in Ethylenvinylacetat (EVA), in Polymilchsäure (PLA) und/oder in PLA-Derivaten, in Polybutylenadipat-Terephthalaten (PBAT), in Polybutylensuccinaten (PBS), in Polyhydroxyalkanoaten (PHA), in Blends, in thermoplastischen Polyurethanen (TPU), in Polyurethan-Elastomeren, in PU-Klebstoffen, in PU-Gießharzen, in PU-Schäumen oder in PU-Beschichtungen für Holz, Leder, Kunstleder und Textilien, als Schutz gegen hydrolytischen Abbau.

14. Verwendung der Carbodiimide nach einem der Ansprüche 1 bis 6 in Folien, insbesondere in Folien für Solarzellen.

15. Folien enthaltend mindestens einen Polyester, ausgewählt aus der Gruppe Polyethylenterephthalat (PET), Ethylenvinylacetat (EVA), Polyethylennaphthalat (PEN), Polybutylenterephthalat (PBT), Polytrimethylene-terephthalat (PTT) und/oder Polycyclohexandimethanol-terephthalat (PCT) und 1,0 - 3,0 Gew.% mindestens eines Carbodiimids nach einem der Ansprüche 1 bis 6, bezogen auf den Polyester.

16. Formmassen aus Polyamid (PA) enthaltend 1,0 - 3,0 Gew.% eines Carbodiimids nach einem der Ansprüche 1 bis 6, bezogen auf das Polyamid, und gegebenenfalls weitere Additive, Füll- und/oder Verstärkungsstoffe.

## Claims

1. Carbodiimide having terminal urea and/or urethane groups of formula (I) 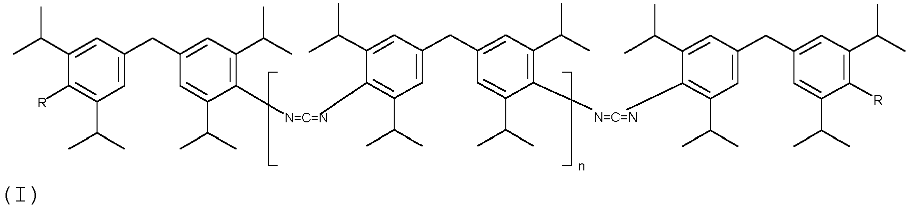
- in which R may be identical or different and is selected from the group of -NHCONHR^{I}, -NHCONR^{I}R^{II} and -NHCOOR^{III} radicals, wherein R^{I} and R^{II} are identical or different and represent a C₁ - C₂₂ - alkyl, C₆ - C₁₂ -cycloalkyl, C₆ - C₁₈ -aryl or C₇ - C₁₈ -aralkyl radical and R^{III} represents a C₁ - C₂₂ - alkyl, C₆ - C₁₂ -cycloalkyl, C₆ - C₁₈ -aryl or C₇ - C₁₈ -aralkyl radical, or an unsaturated alkyl radical having 2-22, preferably 12-20, particularly preferably 16-18, carbon atoms, or an alkoxypolyoxyalkylene radical, and
- n = 0 to 20, preferably n = 1 to 15.

2. Carbodiimide according to Claim 1, **characterized in that** R is an - NHCOOR^{III} radical where R^{III} is an alkoxypolyoxyalkylene or an unsaturated alkyl radical having 18 carbon atoms and n= 0 to 20, preferably n = 1 to 10, particularly preferably n = 2 to 8, very particularly preferably n = 3 to 6.

3. Carbodiimide according to Claim 1, **characterized in that** R is an - NHCOOR^{III} radical, wherein R^{III} is C₁ - C₂₂ - alkyl, preferably C₁ - C₆ - alkyl, particularly preferably methyl, ethyl or i-propyl, C₆ - C₁₂ -cycloalkyl, very particularly preferably C₆ -cycloalkyl, and n = 0 to 15, preferably n = 1 to 15, particularly preferably n = 2 to 10, very particularly preferably n = 3 to 8.

4. Carbodiimide according to Claim 2, **characterized in that** the NCN content in the carbodiimide is 2 - 8 wt%, preferably 3 - 6 wt%, particularly preferably 4 - 5 wt%.

5. Carbodiimide according to Claim 3, **characterized in that** the NCN content in the carbodiimide is 2 - 10 wt%, preferably 3 - 10 wt%, particularly preferably 4 - 8 wt%.

6. Carbodiimide according to any of Claims 1 to 5, **characterized in that** it has an average molar mass (Mw) of 1000 - 10 000 g/mol, preferably 2000 - 8000 g/mol, particularly preferably 3000 - 6000 g/mol.

7. Process for producing the carbodiimides according to any of Claims 1 to 6, **characterized in that** diisocyanates of formula (II) 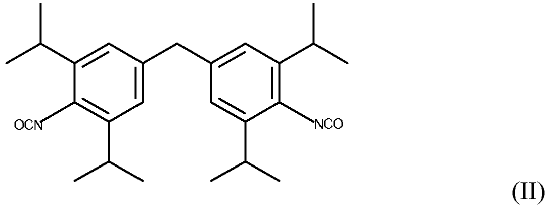 are carbodiimized to eliminate carbon dioxide at temperatures of 80 °C to 200 °C in the presence of catalysts and optionally solvent and subsequently the free NCO functionalities are end-functionalized with primary or secondary amines and/or alcohols.

8. Process for producing the carbodiimides according to any of Claims 1 to 6, **characterized in that** initially a partial, preferably <50%, end-functionalization of the free NCO groups with primary or secondary amines and/or alcohols and/or alkoxypolyoxyalkylene alcohols in the aromatic diisocyanates of formula (II) 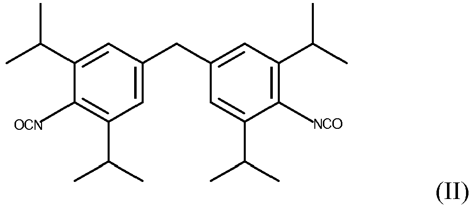 is performed and subsequently a carbodiimidization to eliminate carbon dioxide at temperatures of 80 °C to 200 °C in the presence of catalysts and optionally solvent is effected.

9. Process for producing the carbodiimides according to Claim 3, **characterized in that** after production the melt is pelletized on the pelletizing belts.

10. Composition comprising
- at least one ester-based polymer preferably selected from the group of polyester polyols, ester-based thermoplastic polyurethanes, polyurethane elastomers, PU adhesives, PU casting resins, polyamides (PA), polyethylene terephthalates (PET), polybutylene terephthalates (PBT), polytrimethylene terephthalates (PTT), copolyesters, thermoplastic polyester elastomers (TPE E), ethylene vinyl acetates (EVA), polylactic acids (PLA), polybutylene adipate terephthalates (PBAT), polybutylene succinates (PBS), PLA derivatives and/or polyhydroxyalkanoates (PHA)
and
- at least one carbodiimide according to any of Claims 1 to 6.

11. Composition according to Claim 10, **characterized in that** the concentration of the carbodiimide is 0,1 - 10 wt%, preferably 1 - 5 wt%, particularly preferably 1 - 3 wt%.

12. Process for producing the compositions according to Claim 10 or 11, **characterized in that** carbodiimides according to Claim 3 are added by means of solids metering units to the ester-based polymers selected from the group comprising polyethylene terephthalate (PET), polybutylene terephthalate (PBT), polytrimethylene terephthalate (PTT), polyamides (PA), thermoplastic polyurethanes (TPU), copolyesters, modified polyester made of cyclohexanediol and terephthalic acid (PCTA), thermoplastic polyester elastomers (TPE E), ethylene vinyl acetate (EVA), polylactic acid (PLA), polybutylene adipate terephthalate (PBAT), polybutylene succinate (PBS), PLA derivatives and/or polyhydroxyalkanoates (PHA).

13. Use of the carbodiimides according to any of Claims 1 to 6 in ester-based polyols, in polyamides (PA), in polyethylene terephthalate (PET), in polybutylene terephthalate (PBT), in polytrimethylene terephthalate (PTT), in copolyesters, in thermoplastic polyester elastomers (TPE E), in ethylene vinyl acetate (EVA), in polylactic acid (PLA) and/or in PLA derivatives, in polybutylene adipate terephthalates (PBAT), in polybutylene succinates (PBS), in polyhydroxyalkanoates (PHA), in blends, in thermoplastic polyurethanes (TPU), in polyurethane elastomers, in PU adhesives, in PU casting resins, in PU foams or in PU coatings for wood, leather, synthetic leather and textiles as protection from hydrolytic degradation.

14. Use of the carbodiimides according to any of Claims 1 to 6 in films, in particular in films for solar cells.

15. Film comprising at least one polyester selected from the group polyethylene terephthalate (PET), ethylene vinyl acetate (EVA), polyethylene naphthalate (PEN), polybutylene terephthalate (PBT), polytrimethylene terephthalate (PTT) and/or polycyclohexanedimethanol terephthalate (PCT) and 1.0 - 3.0 wt% of at least one carbodiimide according to any of Claims 1 to 6 based on the polyester.

16. Molding material made of polyamide (PA) comprising 1.0 - 3.0 wt% of a carbodiimide according to any of Claims 1 to 6 based on the polyamide and optionally further additives, fillers and/or reinforcers.

## Revendications

1. Carbodiimides contenant des groupes urée et/ou uréthane terminaux de formule (I) 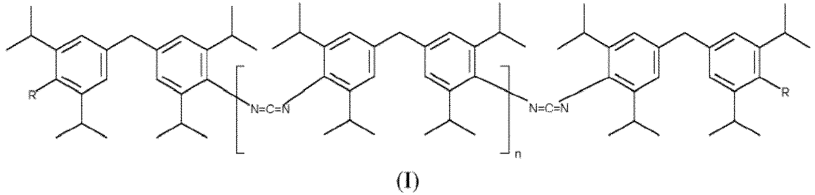
- dans laquelle les R peuvent être identiques ou différents, et sont choisis dans le groupe constitué par les radicaux -NHCONHR^{I}-, -NHCONR^{I}R^{II}- et -NHCOOR^{III}, R^{I} et R^{II} étant identiques ou différents, et correspondant à un radical alkyle en C₁-C₂₂, cycloalkyle en C₆-C₁₂, aryle en C₆-C₁₈ ou aralkyle en C₇-C₁₈, et R^{III} correspondant à un radical alkyle en C₁-C₂₂, cycloalkyle en C₆-C₁₂, aryle en C₆-C₁₈ ou aralkyle en C₇-C₁₈, ou un radical alkyle insaturé de 2 à 22, de préférence 12 à 20, de manière particulièrement préférée 16 à 18 atomes de carbone, ou un radical alcoxypolyoxyalkylène, et
- n = 0 à 20, de préférence n = 1 à 15.

2. Carbodiimides selon la revendication 1, **caractérisés en ce que** R est un radical -NHCOOR^{III} avec R^{III} un alcoxypolyoxyalkylène ou un radical alkyle insaturé de 18 atomes de carbone, et n = 0 à 20, de préférence n = 1 à 10, de manière particulièrement préférée n = 2 à 8, de manière tout particulièrement préférée n = 3 à 6.

3. Carbodiimides selon la revendication 1, **caractérisés en ce que** R est un radical -NHCOOR^{III} avec R^{III} un radical alkyle en C₁-C₂₂, de préférence alkyle en C₁-C₆, de manière particulièrement préférée méthyle, éthyle ou i-propyle, cycloalkyle en C₆-C₁₂, de manière tout particulièrement préférée cycloalkyle en C₆, et n = 0 à 15, de préférence n = 1 à 15, de manière particulièrement préférée n = 2 à 10, de manière tout particulièrement préférée n = 3 à 8.

4. Carbodiimides selon la revendication 2, **caractérisés en ce que** la teneur en NCN dans le carbodiimide est de 2 à 8 % en poids, de préférence de 3 à 6 % en poids, de manière particulièrement préférée de 4 à 5 % en poids.

5. Carbodiimides selon la revendication 3, **caractérisés en ce que** la teneur en NCN dans le carbodiimide est de 2 à 10 % en poids, de préférence de 3 à 10 % en poids, de manière particulièrement préférée de 4 à 8 % en poids.

6. Carbodiimides selon l'une quelconque des revendications 1 à 5, **caractérisés en ce que** ceux-ci présentent des masses molaires moyennes (Mw) de 1 000 à 10 000 g/mol, de préférence de 2 000 à 8 000 g/mol, de manière particulièrement préférée de 3 000 à 6 000 g/mol.

7. Procédé de fabrication des carbodiimides selon l'une quelconque des revendications 1 à 6, **caractérisé en ce que** des diisocyanates de formule (II) 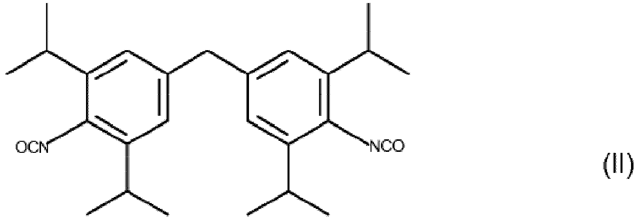 sont carbodiimidés avec clivage de dioxyde de carbone à des températures de 80 °C à 200 °C en présence de catalyseurs et éventuellement de solvants, puis les fonctionnalités NCO libres sont fonctionnalisées en position terminale avec des amines et/ou des alcools primaires ou secondaires.

8. Procédé de fabrication des carbodiimides selon l'une quelconque des revendications 1 à 6, **caractérisé en ce qu'**une fonctionnalisation en position terminale partielle, de préférence à 50 %, des groupes NCO libres avec des amines et/ou des alcools primaires ou secondaires et/ou des alcoxypolyoxyalkylène-alcools en les diisocyanates aromatiques de formule (II) 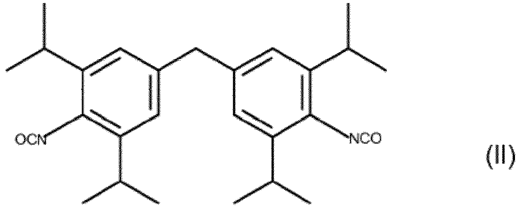 est tout d'abord réalisée, puis une carbodiimidation avec clivage de dioxyde de carbone à des températures de 80 °C à 200 °C en présence de catalyseurs et éventuellement de solvants a lieu.

9. Procédé de fabrication des carbodiimides selon la revendication 3, **caractérisé en ce que** la masse fondue est partillée après la fabrication sur des bandes de pastillage.

10. Composition contenant :
- au moins un polymère à base d'ester, de préférence choisi dans le groupe constitué par les polyester-polyols, les polyuréthanes thermoplastiques à base d'ester, les élastomères de polyuréthane, les adhésifs de PU, les résines de coulée de PU, les polyamides (PA), le polyéthylène-téréphtalate (PET), le polybutylène-téréphtalate (PBT), le polytriméthylène-téréphtalate (PTT), les copolyesters, les polyesters thermoplastiques-élastomères (TPE E), l'éthylène-acétate de vinyle (EVA), les acides polylactiques (PLA), les polybutylène-adipate-téréphtalates (PBAT), les polysuccinates de butylène (PBS), les dérivés de PLA et/ou les polyhydroxyalcanoates (PHA),
et
- au moins un carbodiimide selon l'une quelconque des revendications 1 à 6.

11. Composition selon la revendication 10, **caractérisée en ce que** la concentration du carbodiimide est de 0,1 à 10 % en poids, de préférence de 1 à 5 % en poids, de manière particulièrement préférée de 1 à 3 % en poids.

12. Procédé de fabrication des compositions selon la revendication 10 ou 11, **caractérisé en ce que** des carbodiimides selon la revendication 3 sont ajoutés aux polymères à base d'ester choisis dans le groupe comprenant le polyéthylène-téréphtalate (PET), le polybutylène-téréphtalate (PBT), le polytriméthylène-téréphtalate (PTT), les polyamides (PA), les polyuréthanes thermoplastiques (TPU), les copolyesters, les polyesters modifiés de cyclohexanediol et d'acide téréphtalique (PCTA), les polyesters thermoplastiques-élastomères (TPE E), l'éthylène-acétate de vinyle (EVA), l'acide polylactique (PLA), les polybutylène-adipate-téréphtalates (PBAT), les polysuccinates de butylène (PBS), les dérivés de PLA et/ou les polyhydroxyalcanoates (PHA), au moyen d'un appareil de dosage de solides.

13. Utilisation des carbodiimides selon l'une quelconque des revendications 1 à 6 dans des polyols à base d'ester, dans des polyamides (PA), dans du polyéthylène-téréphtalate (PET), dans du polybutylène-téréphtalate (PBT), dans du polytriméthylène-téréphtalate (PTT), dans des copolyesters, dans des polyesters thermoplastiques-élastomères (TPE E), dans de l'éthylène-acétate de vinyle (EVA), dans de l'acide polylactique (PLA) et/ou dans des dérivés de PLA, dans des polybutylène-adipate-téréphtalates (PBAT), dans des polysuccinates de butylène (PBS), dans des polyhydroxyalcanoates (PHA), dans des mélanges, dans des polyuréthanes thermoplastiques (TPU), dans des élastomères de polyuréthane, dans des adhésifs de PU, dans des résines de coulée de PU, dans des mousses de PU ou dans des revêtements de PU pour le bois, le cuir, le cuir artificiel et les textiles, en tant que protection contre la décomposition hydrolytique.

14. Utilisation des carbodiimides selon l'une quelconque des revendications 1 à 6 dans des films, notamment dans des films pour cellules solaires.

15. Films contenant au moins un polyester, choisi dans le groupe constitué par le polyéthylène-téréphtalate (PET), l'éthylène-acétate de vinyle (EVA), le polyéthylène-naphtalate (PEN), le polybutylène-téréphtalate (PBT), le polytriméthylène-téréphtalate (PTT) et/ou le polycyclohexanediméthanol-téréphtalate (PCT) et 1,0 à 3,0 % en poids d'au moins un carbodiimide selon l'une quelconque des revendications 1 à 6, par rapport aux polyesters.

16. Matériaux de moulage à base de polyamide (PA) contenant 1,0 à 3,0 % en poids d'un carbodiimide selon l'une quelconque des revendications 1 à 6, par rapport au polyamide, et éventuellement d'autres additifs, charges et/ou matières de renforcement.
